(19) Europäisches Patentamt
European Patent Office
Office européen des brevets

(11) Veröffentlichungsnummer: **0 346 727 A1**

(12) **EUROPÄISCHE PATENTANMELDUNG**

(21) Anmeldenummer: **89110189.1**

(22) Anmeldetag: **06.06.89**

(51) Int. Cl.4: **C07D 233/56 , C07D 249/08 , A01N 43/50 , A01N 43/653**

(30) Priorität: **11.06.88 DE 3819903**

(43) Veröffentlichungstag der Anmeldung: **20.12.89 Patentblatt 89/51**

(84) Benannte Vertragsstaaten: **AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Seele, Rainer, Dr.**
**Leiblstrasse 3**
**D-6701 Fussgoenheim(DE)**
Erfinder: **Himmele, Walter, Dr.**
**Eichenweg 14**
**D-6909 Walldorf(DE)**
Erfinder: **Goetz, Norbert, Dr.**
**Schoefferstrasse 25**
**D-6520 Worms 1(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

(54) **1-Halogen-1-azolyl-ethan-derivate und diese enthaltende Fungizide.**

(57) 1-Chlor-1-azolyl-ethan-derivate der allgemeinen Formel I

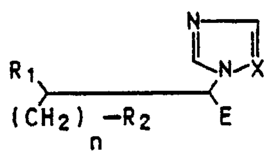

in welcher $R^1$ und $R^2$ Alkyl, Phenyl, Biphenyl, Naphthyl, Benzyl, Cycloalkyl und Cycloalkenyl bedeuten, wobei diese Reste gegebenenfalls substituiert sind,
n eine ganze Zahl von 1 bis 5 oder 0 bedeutet,
E den Rest Cl oder Br bedeutet,
X CH oder N bedeutet
und deren für Pflanzen verträgliche Säureadditionssalze und Metallkomplexe und diese Verbindungen enthaltende Fungizide.

EP 0 346 727 A1

## 1-Halogen-1-azolyl-ethan-derivate und diese enthaltende Fungizide

Die vorliegende Erfindung betrifft neue Halogen-Azolverbindungen, Verfahren zu ihrer Herstellung und diese enthaltende Fungizide.

Es ist bekannt, 1-(1,2,4-Triazol-1-yl)-2-(2,4-dichlorphenyl)-pentan als Fungizid zu verwenden (Thomson, Agricultural Chemicals, Book IV, Fungicides, page 124, 1988).

Es wurde nun gefunden, daß 1-Halogen-1-azolyl-ethan-derivate der allgemeinen Formel I

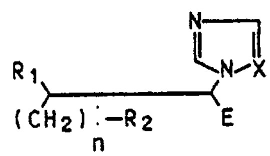

in welcher $R^1$ und $R^2$ $C_1$-$C_8$-Alkyl, Phenyl, Biphenyl, Naphthyl, Benzyl, $C_3$-$C_8$-Cycloalkyl und $C_3$-$C_8$-Cycloalkenyl bedeuten, wobei diese Reste gegebenenfalls durch Halogen, Nitro, Phenoxy, Alkyl, Alkoxy, Amino oder Halogenalkyl mit jeweils 1- bis 4-C-Atomen substituiert sind,
n eine ganze Zahl von 1 bis 5 oder 0 bedeutet,
E den Rest Cl oder Br bedeutet,
X CH oder N bedeutet
sowie deren für Pflanzen verträgliche Säureadditionssalze und Metallkomplexe eine bessere fungizide Wirkung, insbesondere gegen Getreidekrankheiten, besitzen als bekannte Azolverbindungen.

Die Verbindungen der Formel I enthalten asymmetrische C-Atome und können daher als Enantiomere und Diastereomere auftreten. Die Erfindung umfaßt sowohl die reinen Isomeren als auch deren Gemische. Die Gemische von Diastereomeren können nach bekannten Methoden, zum Beispiel durch fraktionierte Kristallisation oder durch Chromatographie an Kieselgel in die Komponenten getrennt werden. Die Racemate lassen sich bei den erfindungsgemäßen Verbindungen mit üblichen Methoden, beispielsweise durch Salzbildung mit einer optisch aktiven Säure, Trennung der diastereomeren Salze und Freisetzung der Enantiomeren mittels einer Base, trennen.

Als fungizide Wirkstoffe können sowohl die einzelnen Enantiomeren oder Diastereomeren als auch deren Gemische verwendet werden.

$R^1$ und $R^2$ sind gleich oder verschieden und bedeuten beispielsweise:
Methyl, Ethyl, Isopropyl, n-Propyl, n-Butyl, sec-Butyl, tert.-Butyl, n-Pentyl, Neopentyl, Hexyl, Octyl, Trifluormethyl, Trichlormethyl, Phenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, 2-Methoxyphenyl, 3-Methoxyphenyl, 4-Methoxyphenyl, 2,4-Dimethoxyphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Trifluoromethylphenyl, 3-Trifluoromethylphenyl, 4-Trifluoromethylphenyl, Cyclopropyl, Cyclobutyl, Cyclopentyl, Cyclohexyl, Cyclooctyl, $C_6$-Cycloalkenyl, 2-Cyclohexenyl, 3-Cyclohexenyl, Norbornyl.

n bedeutet 0, 1, 2, 3, 4 oder 5.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion i. a. nicht ankommt. Die erfindungsgemäßen Wirkstoffsalze werden hergestellt durch Umsetzung der 1-Halogen-1-azolylethan-derivate (I) mit den Säuren.

Metallkomplexe der Wirkstoffe I oder ihrer Salze können mit Kupfer, Zink, Zinn, Mangan, Eisen, Kobalt oder Nickel gebildet werden, indem man die 1-Halogen-1-azolylethan-derivate mit entsprechenden Metallsalzen umsetzt, z.B. mit Kupfersulfat, Zinkchlorid, Zinnchlorid.

Die Verbindungen der Formel I können z. B. hergestellt werden, indem man eine Verbindung der Formel II

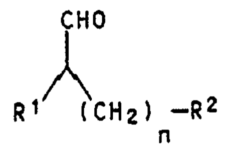

in welcher $R^1$, $R^2$ und n die angegebenen Bedeutungen haben, mit einer Verbindung der Formel III,

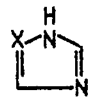

in der X die angegebene Bedeutung hat, in Gegenwart von entsprechenden Thionylhalogeniden zur Umsetzung bringt.

Die Reaktion erfolgt gegebenenfalls in Gegenwart eines Lösungs-oder Verdünnungsmittels bei Temperaturen zwischen -30 und 80° C. Zu den bevorzugten Lösungs- und Verdünngungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Toluol, Methylenchlorid, Chloroform, Tetrachlorkohlenstoff, Dichlorethan oder entsprechende Gemische.

Die Verbindungen der Formel II lassen sich entsprechend allgemein bekannten Verfahren zur Aldehydsynthese (Houben-Weyl-Müller, Methoden der organischen Chemie, Georg Thieme Verlag, Stuttgart 1983, Bd E 3) herstellen.

Das nachfolgende Beispiel erläutert die Herstellung der Wirkstoffe.

Beispiel 1

Zu einer Lösung von 15,2 g 1,2,4-Triazol in 150 ml Methylenchlorid werden bei 0° C 13,1 g Thionylchlorid zugesetzt. Nach beendeter Zugabe wird die Mischung bei Raumtemperatur (20° C) für 30 Minuten gerührt und anschließend 15,5 g 2,3-Biphenylpropanal zugegeben. Nachdem das Reaktionsgemisch 12-15 Stunden bei Raumtemperatur rührte, werden der Lösung 100 ml Wasser zugesetzt und die organische Phase abgetrennt. Die verbleibende wäßrige Phase wird zweimal mit Methylenchlorid ausgeschüttelt und die gesammelten organischen Phasen zweimal mit gesättigter Natriumhydrogencarbonat-Lösung gewaschen. Die isolierte organische Phase wird daraufhin über Natriumsulfat getrocknet und eingeengt, wobei 30,0 g (92 %) 1-Chlor-1-(1,2,4-triazol-1-yl)-2,3-diphenyl-propan erhalten werden.

Entsprechend Beispiel 1 können die in der Tabelle aufgeführten Verbindungen hergestellt werden.

Tabelle:

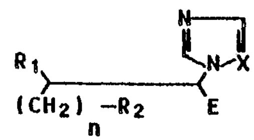

| Bsp. | $R^1$ | $R^2$ | n | E | X | D1:D2* | IR ($cm^{-1}$) |
|---|---|---|---|---|---|---|---|
| 1 | $C_6H_5$ | $C_6H_5$ | 1 | Cl | N | 4:1 | 1506,1497,1275,1135,762,699 |
| 2 | $C_6H_5$ | 4-F-$C_6H_4$ | 1 | Cl | N | - | - |
| 3 | $C_6H_5$ | 2-F-$C_6H_4$ | 1 | Cl | N | - | - |
| 4 | $C_6H_5$ | 2-Cl-$C_6H_4$ | 1 | Cl | N | - | - |
| 5 | $C_6H_5$ | 4-Cl-$C_6H_4$ | 1 | Cl | N | - | - |
| 6 | $C_6H_5$ | 2,4-$Cl_2$-$C_6H_3$ | 1 | Cl | N | - | - |
| 7 | $C_6H_5$ | 2,4-$Cl_2$-$C_6H_3$ | 1 | Cl | CH | - | - |
| 8 | $C_6H_5$ | $C_{10}H_7$ | 1 | Cl | N | - | - |
| 9 | $C_6H_5$ | $CH_3$ | 1 | Cl | N | - | - |
| 10 | $C_6H_5$ | $C_4H_9$ | 1 | Cl | N | - | - |
| 11 | $C_6H_5$ | Cyclohexyl | 1 | Cl | N | - | - |
| 12 | $C_6H_5$ | $C_6H_5$ | 0 | Cl | N | 1:1 | 1506,1499,1275,1134,742,703 |
| 13 | $C_6H_5$ | 4-F-$C_6H_5$ | 0 | Cl | N | - | - |
| 14 | $C_6H_5$ | 2-Cl-$C_6H_5$ | 0 | Cl | N | - | - |
| 15 | $C_6H_5$ | $C_{12}H_9$ | 0 | Cl | N | - | - |
| 16 | $C_6H_5$ | Cyclohexyl | 0 | Cl | N | 1:1 | 2927,2854,1505,1450,1275,1135,703 |
| 17 | $C_6H_5$ | 3-Cyclohexenyl | 0 | Cl | N | - | - |
| 18 | $C_6H_5$ | $C_6H_5$ | 2 | Cl | N | 3:1 | 1506,1496,1276,1135,750,700 |
| 19 | $C_6H_5$ | 4-F-$C_6H_5$ | 2 | Cl | N | - | - |
| 20 | $C_6H_5$ | 2-Cl-$C_6H_5$ | 2 | Cl | N | - | - |
| 21 | $C_6H_5$ | $C_6H_5$ | 3 | Cl | N | - | - |

Tabelle (Fortsetzung)

| Bsp. | $R^1$ | $R^2$ | n | E | X | D1:D2* | IR ($cm^{-1}$) |
|---|---|---|---|---|---|---|---|
| 22 | $C_6H_5$ | 4-Cl-$C_6H_4$ | 3 | Cl | N | - | - |
| 23 | 4-F-$C_6H_4$ | $C_6H_5$ | 0 | Cl | N | - | - |
| 24 | 4-F-$C_6H_4$ | 4-F-$C_6H_4$ | 0 | Cl | N | - | 1508,1229,1160,837 |
| 25 | 4-F-$C_6H_4$ | 2-F-$C_6H_4$ | 0 | Cl | N | 1:1 | 1510,1493,1276,1233,809,756 |
| 26 | 4-F-$C_6H_4$ | 2-Cl-$C_6H_4$ | 0 | Cl | N | - | - |
| 27 | 4-F-$C_6H_4$ | 4-Cl-$C_6H_4$ | 0 | Cl | N | - | - |
| 28 | 4-F-$C_6H_4$ | 2,4-$Cl_2$-$C_6H_3$ | 0 | Cl | N | - | - |
| 29 | 4-F-$C_6H_4$ | 4-$OCH_3$-$C_6H_4$ | 0 | Cl | N | - | - |
| 30 | 4-F-$C_6H_4$ | iso-$C_3H_7$ | 0 | Cl | N | 7:3 | 2959,1510,1275,1229,1136,841 |
| 31 | 4-F-$C_6H_4$ | 2-F-$C_6H_4$ | 1 | Cl | N | - | - |
| 32 | 4-F-$C_6H_4$ | 4-F-$C_6H_4$ | 2 | Cl | N | - | - |
| 33 | 2-Cl-$C_6H_4$ | 4-F-$C_6H_4$ | 0 | Cl | N | - | - |
| 34 | 2-Cl-$C_6H_4$ | 4-Cl-$C_6H_4$ | 0 | Cl | N | - | - |
| 35 | 2-Cl-$C_6H_4$ | 2,4-$Cl_2$-$C_6H_3$ | 1 | Cl | N | - | - |
| 36 | 2-Cl-$C_6H_4$ | $CH_3$ | 1 | Cl | N | - | - |
| 37 | 2-Cl-$C_6H_4$ | $CH_3$ | 2 | Cl | N | 1:1 | 2960,1506,1275,1135,753 |
| 38 | 4-Cl-$C_6H_4$ | 4-Cl-$C_6H_4$ | 0 | Cl | N | - | - |
| 39 | 4-Cl-$C_6H_4$ | 4-F-$C_6H_4$ | 1 | Cl | N | - | - |
| 40 | 4-Cl-$C_6H_4$ | $CH_3$ | 1 | Cl | N | - | - |
| 41 | 4-Cl-$C_6H_4$ | $CH_3$ | 2 | Cl | N | 2:1 | Harz |
| 42 | 2,4-$Cl_2$-$C_6H_3$ | 4-F-$C_6H_4$ | 0 | Cl | N | - | - |
| 43 | 2,4-$Cl_2$-$C_6H_3$ | 2-Cl-$C_6H_4$ | 0 | Cl | N | - | - |
| 44 | 2,4-$Cl_2$-$C_6H_3$ | $CH_3$ | 1 | Cl | N | 1,2:1 | 2970,1506,1476,1275,1199,1135,805 |
| 45 | 2,4-$Cl_2$-$C_6H_3$ | $CH_3$ | 2 | Cl | N | 1:1 | 2961,1506,1475,1275,1199,1135,797 |
| 46 | 2,4-$Cl_2$-$C_6H_3$ | $CH_3$ | 3 | Cl | N | 9:1 | 2958,1506,1475,1275,1135,804 |
| 47 | Cyclohexyl | 4-F-$C_6H_4$ | 1 | Cl | N | - | - |

Tabelle (Fortsetzung)

| Bsp. | $R^1$ | $R^2$ | n | E | X | D1:D2* | IR ($cm^{-1}$) |
|---|---|---|---|---|---|---|---|
| 48 | Cyclohexyl | 2-Cl-$C_6H_4$ | 1 | Cl | N | - | - |
| 49 | Cyclohexyl | 2,4-$Cl_2$-$C_6H_3$ | 1 | Cl | N | - | - |
| 50 | Cyclohexyl | 4-Cl-$C_6H_4$ | 2 | Cl | N | - | - |
| 51 | $CH_3$ | 4-F-$C_6H_4$ | 0 | Cl | N | - | - |
| 52 | $CH_3$ | 2-Cl-$C_6H_4$ | 0 | Cl | N | - | - |
| 53 | $CH_3$ | 4-Cl-$C_6H_4$ | 1 | Cl | N | 1:1 | 1506,1492,1275,1136,1015,811 |
| 54 | $CH_3$ | 4-F-$C_6H_4$ | 1 | Cl | N | 2:1 | 1511,1276,1223,1136,838 |
| 55 | $CH_3$ | 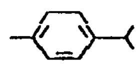 | 1 | Cl | N | 1:1 | 2961,2934,1508,1276,1136,1004,818 |
| 56 | $CH_3$ | 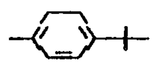 | 1 | Cl | N | - | - |
| 57 | $CH_3$ | 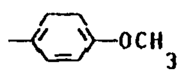 | 1 | Cl | N | 2:1 | 1514,1275,1255,1033,833,768 |
| 58 | $CH_3$ | 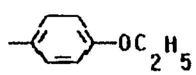 | 1 | Cl | N | 1:1 | 2979,1611,1511,1275,1248,1047 |
| 59 | $CH_3$ | 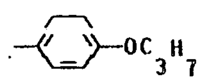 | 1 | Cl | N | 1:1 | 2966,1611,1511,1275,1250,1135 |
| 60 | $CH_3$ | 4-$NO_2$-$C_6H_4$ | 2 | Cl | N | - | - |
| 61 | $CH_3$ | 2-F-$C_6H_4$ | 3 | Cl | N | - | - |
| 62 | $CH_3$ | $CH(C_6H_5)_2$ | 0 | Cl | N | 1:1 | 1496,1451,1277,1137,748,705 |
| 63 | tert.-Butyl | 4-Cl-$C_6H_4$ | 0 | Cl | N | - | - |
| 64 | tert.-Butyl | 4-F-$C_6H_4$ | 0 | Cl | N | - | - |

Tabelle (Fortsetzung)

| Bsp. | $R^1$ | $R^2$ | n | E | X | D1:D2* | IR ($cm^{-1}$)/Schmp. |
|---|---|---|---|---|---|---|---|
| 65 | tert.-Butyl | 2-Cl-$C_6H_4$ | 0 | Cl | N | - | - |
| 66 | tert.-Butyl | 2,4-$Cl_2$-$C_6H_3$ | 0 | Cl | N | - | - |
| 67 | tert.-Butyl | 4-Cl-$C_6H_4$ | 1 | Cl | N | - | - |
| 68 | tert.-Butyl | 4-F-$C_6H_4$ | 1 | Cl | N | - | - |
| 69 | tert.-Butyl | 4-Cl-$C_6H_4$ | 2 | Cl | N | - | - |
| 70 | tert.-Butyl | 4-Cl-$C_6H_4$ | 3 | Cl | N | - | - |
| 71 | tert.-Butyl | Cyclohexyl | 0 | Cl | N | - | - |
| 72 | tert.-Butyl | Cyclohexyl | 1 | Cl | N | - | - |
| 73 | tert.-Butyl | Cyclohexyl | 1 | Cl | N | - | - |
| 74 | 2,4-$Cl_2$-$C_6H_3$ | $CH_3$ | 1 | Cl | CH | 1:1 | 82 - 88° |
| 75 | 2,4-$Cl_2$-$C_6H_3$ | $CH_3$ | 2 | Cl | CH | 1:1 | 78 - 82° |
| 76 | $C_6H_5$ | $C_6H_5$ | 0 | Br | N | - | - |
| 77 | $C_6H_5$ | $C_6H_5$ | 1 | Br | N | 1:1 | 1506,1497,1454,1274,1133,700 |
| 78 | $C_6H_5$ | $C_6H_5$ | 2 | Br | N | - | - |
| 79 | $C_6H_5$ | Cyclohexyl | 0 | Br | N | - | - |
| 80 | 4-F-$C_6H_4$ | Iso-$C_3H_7$ | 0 | Br | N | - | - |
| 81 | 2,4-$Cl_2$-$C_6H_3$ | $CH_3$ | 1 | Br | N | - | - |
| 82 | 2,4-$Cl_2$-$C_6H_3$ | $CH_3$ | 2 | Br | N | 1:1 | 2961,2935,1506,1475,1274,1133,823 |
| 83 | $CH_3$ | 4-F-$C_6H_4$ | 0 | Br | N | - | - |
| 84 | $CH_3$ | 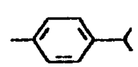 | 1 | Br | N | 1:1 | 2963,2933,2507,1421,1274,1134,817 |
| 85 | $CH_3$ | 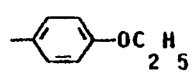 | 1 | Br | N | 1:1 | 2979,1611,1511,1249,1047 |
| 86 | $CH_3$ | 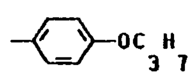 | 1 | Br | N | 1:1 | 2965,1611,1511,1250,1134 |

Tabelle (Fortsetzung)

| Bsp. | $R^1$ | $R^2$ | n | E | X | D1:D2* | IR ($cm^{-1}$)/Schmp. |
|---|---|---|---|---|---|---|---|
| 87 | $CH_3$ | $-CH(C_6H_5)_2$ | 0 | Br | N | - | - |
| 88 | $-C(CH_3)_3$ | $4-Cl-C_6H_4$ | 0 | Br | N | - | - |
| 89 | $C_6H_5$ | $CH_3$ | 0 | Cl | N | 3:1 | Harz |
| 90 | $C_6H_5$ | $CH_3$ | 2 | Cl | N | 3:1 | 2960,1506,1275,1135,700 |
| 91 | $C_6H_5$ | $CH_3$ | 2 | Cl | CH | 1,2:1 | 2959,1229,1153,1088,754 |
| 92 | $4-F-C_6H_4$ | $CH_3$ | 3 | Cl | N | 2:1 | 2958,1510,1276,1226,840 |
| 93 | $4-Cl-C_6H_4$ | $C_2H_5$ | 5 | Cl | N | 2:1 | 2927,1505,1492,1275,1135,1015 |
| 94 | $4-F-C_6H_4$ | $2-F-C_6H_4$ | 0 | Cl | CH | 1:1 | 1511,1491,1230,1078,807,756 |
| 95 | $4-Cl-C_6H_4$ | $CH_3$ | 0 | Cl | N | 3:1 | 1506,1495,1275,1135,1015,836,777 |
| 96 | $4-Cl-C_6H_4$ | $CH_3$ | 2 | Cl | CH | 2:1 | 2960,1492,1092,1015,778 |
| 97 | $4-Cl-C_6H_4$ | $CH_3$ | 5 | Cl | N | 2:1 | 2929,1506,1492,1275,1135,1015,765 |
| 98 | $2,4-Cl_2-C_6H_3$ | $CH_3$ | 2 | Cl | N | 2:1 | 2958,1506,1475,1275,1135,804 |
| 99 | $2,4-Cl_2-C_6H_3$ | $CH_3$ | 2 | Cl | CH | 3:7 | 2957,1489,1478,1226,1076,802 |
| 100 | $2-Cl-C_6H_4$ | $CH_3$ | 5 | Cl | CH | 2:1 | Harz |
| 101 | $3-Cl-C_6H_4$ | $CH_3$ | 2 | Cl | N | 1:1 | 2961,1506,1275,1135,1001,767 |
| 102 | $3,4-Cl_2-C_6H_3$ | $CH_3$ | 1 | Cl | N | 1:1 | 1506,1472,1275,1134,778,776 |
| 103 | $4-Cl-C_6H_4$ | $2-Cl-C_6H_4$ | 0 | Cl | N | 1:1 | 1506,1493,1275,1133,1015,754 |
| 104 | $4-Cl-C_6H_4$ | $2-Cl-C_6H_4$ | 0 | Cl | CH | 1:1 | 1492,1227,1091,1015,754,736 |
| 105 | $2,4-Cl_2-C_6H_3$ | $CH_3$ | 5 | Cl | N | 1:1 | 1506,1475,1275,1135,801,791 |
| 106 | $2,4-Cl_2-C_6H_3$ | $C_3H_7$ | 5 | Cl | N | 1:1 | 2926,1505,1475,1275,1134,825 |
| 107 | $2-Cl-C_6H_4$ | $C_2H_5$ | 5 | Cl | N | 1:1 | 2927,1505,1275,1135,752 |
| 108 | $4-C_6H_5O-C_6H_4$ | $CH_3$ | 5 | Cl | N | 7:2 | 2929,1506,1489,1241,872 |
| 109 | $4-C_6H_5O-C_6H_4$ | $CH_3$ | 1 | Cl | N | 2:1 | 1589,1506,1489,1239,872 |

Tabelle (Fortsetzung)

| Bsp. | $R^1$ | $R^2$ | n | E | X | D1:D2* | IR ($cm^{-1}$) |
|---|---|---|---|---|---|---|---|
| 110 | 2-Naphthyl | $CH_3$ | 1 | Cl | N | 1,2:1 | 1506,1275,1135,780 |
| 111 | 2-Naphthyl | $CH_3$ | 3 | Cl | N | 1:1 | 2957,1506,1275,1135,778 |
| 112 | 2-Naphthyl | $CH_3$ | 5 | Cl | N | 1:1 | 2928,1506,1275,1135,777 |
| 113 | p-$C_6H_5$-$C_6H_4$ | $CH_3$ | 1 | Cl | N | - | - |
| 114 | 4-F-$C_6H_4$ | $C_3H_7$ | 5 | Cl | N | 1:1 | 2927,1510,1276,1226,1135,840 |
| 115 | 2,4-$Cl_2$-$C_6H_3$ | $CH_3$ | 3 | Cl | CH | 2:1 | Harz |
| 116 | $CH_3$ | 4-$OCH_3$-$C_6H_4$ | 1 | Cl | N | 3:1 | 1512,1250,1035,819 |
| 117 | 4-F-$C_6H_4$ | 2-Cl-$C_6H_4$ | 0 | Cl | N | 1:1 | 1509,1276,1233,1059,808,750 |
| 118 | 4-F-$C_6H_4$ | 2-Cl-$C_6H_4$ | 0 | Cl | CH | 1:1 | Harz |
| 119 | 4-F-$C_6H_4$ | $C_6H_4$ | 0 | Cl | N | 1:1 | 1510,1276,1227,1135,809,732 |
| 120 | 4-F-$C_6H_4$ | $C_6H_4$ | 0 | Cl | CH | 1:1 | 1602,1510,1496,1228,813,730 |
| 121 | 4-F-$C_6H_4$ | 4-Cl-$C_6H_4$ | 0 | Cl | N | - | 112°C |

* Mengenverhältnis der hergestellten Diastereomeren

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis,

Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für Zubereitungen sind:

I. Man vermischt 90 Gew.-Teile der Verbindung Nr. 74 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 89 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 94 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 116 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 74 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 89 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 94 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 116 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 74 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,

N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiel

Als Vergleichswirkstoff wurde 1-(1,2,4-Triazol-1-yl)-2-(2,4-dichlorphenyl)-pentan (A) - bekannt aus Thomson, Agricultural Chemicals, Book IV, Fungicides, page 124, 1988) - verwendet.

Anwendungsbeispiel

Wirksamkeit gegen Botrytis cinerea an Paprika

Paprikaschämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 74, 89, 94 und 116 bei der Anwendung als 0,05 %ige (Gew.%) Spritzbrühe eine bessere fungizide Wirkung zeigen (97 %) als der bekannte Vergleichswirkstoff A (60 %).

## Ansprüche

1. 1-Halogen-1-azolyl-ethan-derivate der allgemeinen Formel I

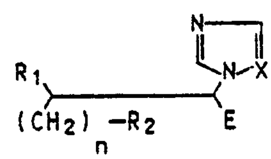

in welcher $R^1$ und $R^2$ $C_1$-$C_8$-Alkyl, Phenyl, Biphenyl, Naphthyl, Benzyl, $C_3$-$C_8$-Cycloalkyl und $C_3$-$C_8$-Cycloalkenyl bedeuten, wobei diese Reste gegebenenfalls durch Halogen, Nitro, Phenoxy, Alkyl, Alkoxy, Amino oder Halogenalkyl mit jeweils 1- bis 4-C-Atomen substituiert sind,
n eine ganze Zahl von 1 bis 5 oder 0 bedeutet,
E den Rest Cl oder Br bedeutet,
X CH oder N bedeutet
sowie deren für Pflanzen verträgliche Säureadditionssalze oder Metallkomplexe.

2. Verfahren zur Herstellung der 1-Halogen-1-azolyl-ethan-derivate der Formel I gemäß Anspruch 1, dadurch gekennzeichnet, daß man eine Verbindung der Formel II

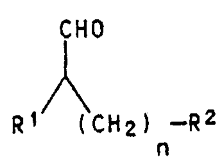

in welcher $R^1$, $R^2$ und n die oben angegebenen Bedeutungen haben, mit einer Verbindung der Formel III,

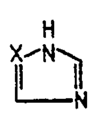

in der X die oben angegebene Bedeutung hat, in Gegenwart von entsprechenden Thionylhalogeniden zur Umsetzung bringt und die so erhaltenen Verbindungen gegebenenfalls in ihre für Pflanzen verträglichen Säureadditionssalze oder Metallkomplexe überführt.

3. Fungizides Mittel, enthaltend eine fungizid wirksame Menge eines 1-Halogen-1-azolyl-ethan-derivats der allgemeinen Formel I

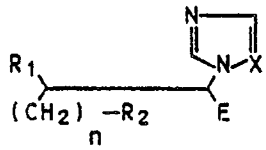

in welcher $R^1$ und $R^2$ $C_1$-$C_8$-Alkyl, Phenyl, Biphenyl, Naphthyl, Benzyl, $C_3$-$C_8$-Cycloalkyl und $C_3$-$C_8$-Cycloalkenyl bedeuten, wobei diese Reste gegebenenfalls durch Halogen, Nitro, Phenoxy, Alkyl, Alkoxy, Amino oder Halogenalkyl mit jeweils 1- bis 4-C-Atomen substituiert sind,
n eine ganze Zahl von 1 bis 5 oder 0 bedeutet,
E den Rest Cl oder Br bedeutet,
X CH oder N bedeutet,
oder dessen für Pflanzen verträgliches Säureadditionssalz oder Metallkomplex und einen inerten Zusatzstoff.

4. Verfahren zur Bekämpfung von Pilzen, dadurch gekennzeichnet, daß man eine fungizid wirksame Menge eines 1-Halogen-1-azolyl-ethan-derivates der allgemeinen Formel I

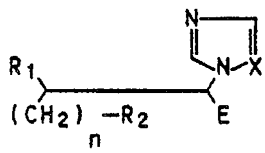

in welcher $R^1$ und $R^2$ $C_1$-$C_8$-Alkyl, Phenyl, Biphenyl, Naphthyl, Benzyl, $C_3$-$C_8$-Cycloalkyl und $C_3$-$C_8$-Cycloalkenyl bedeuten, wobei diese Reste gegebenenfalls durch Halogen, Nitro, Phenoxy, Alkyl, Alkoxy, Amino oder Halogenalkyl mit jeweils 1- bis 4-C-Atomen substituiert sind,
n eine ganze Zahl von 1 bis 5 oder 0 bedeutet,
E den Rest Cl oder Br bedeutet,
X CH oder N bedeutet
oder dessen für Pflanzen verträgliches Säureadditionssalz- oder Metallkomplex auf die Pilze oder durch Pilzbefall bedrohte Materialien, Flächen, Pflanzen oder Saatgüter einwirken läßt.

5. Verbindung der Formel I gemäß Anspruch 1, in der $R^1$ Phenyl, $R^2$ Methyl, E Chlor und X N bedeutet und n den Wert 0 hat.

6. Verbindung der Formel I gemäß Anspruch 1, in der $R^1$ 4-Fluorphenyl, $R^2$ 2-Fluorphenyl, E Chlor und X CH bedeutet und n den Wert 0 hat.

7. Verbindung der Formel I gemäß Anspruch 1, in der $R^1$ 2,4-Dichlorphenyl, $R^2$ Methyl, E Chlor und X CH bedeutet und n den Wert 1 hat.

8. Verbindung der Formel I gemäß Anspruch 1, in der $R^1$ 2,4-Dichlorphenyl, $R^2$ Methyl, E Chlor und X CH bedeutet und n den Wert 3 hat.

Europäisches Patentamt

# EUROPÄISCHER RECHERCHENBERICHT

Nummer der Anmeldung

EP 89 11 0189

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 163 606 (CIBA-GEIGY)<br>* Ansprüche 1,13 *<br>----- | 1,3 | C 07 D 233/56<br>C 07 D 249/08<br>A 01 N 43/50<br>A 01 N 43/653 |
| | | | **RECHERCHIERTE SACHGEBIETE (Int. Cl.4)** |
| | | | C 07 D 233/00<br>C 07 D 249/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 01-09-1989 | CASADO Y MARTIN DE MERCA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument